**Europäisches Patentamt**

**European Patent Office**

**Office européen des brevets**

(19)

(11) Veröffentlichungsnummer: **0 603 881 A2**

(12) **EUROPÄISCHE PATENTANMELDUNG**

(21) Anmeldenummer: **93120786.4**

(22) Anmeldetag: **23.12.93**

(51) Int. Cl.⁵: **C12Q 1/68**, G01N 33/14,
//(C12Q1/68,C12R1:01)

(30) Priorität: **24.12.92 DE 4244167**

(43) Veröffentlichungstag der Anmeldung:
**29.06.94 Patentblatt 94/26**

(84) Benannte Vertragsstaaten:
**AT CH DE ES FR GR IT LI PT**

Bahnhofstr.46
74348 Lauffen a.N.(DE)
Erfinder: **Otten, Léon Prof. Dr., IBMB du CNRS**
**12, rue du Général Zimmer**
**67000 Strasbourg(FR)**

(71) Anmelder: **Schulz, Thomas F., Dr.**
**Bahnhostrasse 46**
**D-74348 Lauffen(DE)**

(72) Erfinder: **Schulz, Thomas F. DR.**

(74) Vertreter: **Zeitler & Dickel**
**Patentanwälte**
**European Patent Attorneys**
**Postfach 26 02 51**
**D-80059 München (DE)**

(54) **Verfahren zum Nachweis von Agrobacterium vitis in Weinreben.**

㊛ Bei einem Verfahren zum Nachweis von Agrobacterium vitis in Weinreben werden T-DNA-Fragmente des Pathogens in infizierten Weinreben mittels Polymerase-Kettenreaktion (PCR) nachgewiesen. Hierbei werden spezifische DNA-Fragmente von Tumorgenen und/oder Insertionselementen mittels der Polymerase-Kettenreaktion amplifiziert und anschließend durch DNA-Gelelektrophorese identifiziert.

Fig. 1

Die Erfindung betrifft ein Verfahren zum Nachweis von Agrobacterium vitis in Mauke-infizierten Weinreben.

Die durch Agrobacterium vitis verursachte Mauke in Weinreben hat weltweit zu einer starken Beeinträchtigung des Weinbaues geführt. Sie äußert sich in krebsartigen Geschwulsten, die insbesondere an den Veredelungsstellen erscheinen, sich aber auch leistenförmig über den ganzen Stamm bis zur Tragrute hin erstrecken können. Auch an unterirdischen Teilen der Unterlagsrebe treten durch Mauke verursachte Schadensbilder auf.

Insbesondere die Unkenntnis über die latente Infektion mit Agrobacterium vitis in Weinreben hatte zur Folge, daß lange Zeit mit diesem Pathogen verseuchtes Pflanzenmaterial vermehrt wurde, was zur weltweiten Verbreitung der Mauke maßgeblich beigetragen hat.

Man hat bereits auf unterschiedliche Weise versucht, Agrobacterium vitis zu bestimmen, um dadurch frühzeitig Behandlungsverfahren einleiten zu können und eine Vermehrung kranken Materials zu vermeiden.

Ein Verfahren liegt in der Bestimmung von Agrobacterium vitis anhand physiologischer Merkmale. Die Abgrenzung ist jedoch nicht immer eindeutig, wodurch die Zuordnung der Bakterien oft erschwert wird. Für ein routinemäßiges Testen ist dieses Verfahren zu zeitaufwendig.

Es wurde auch bereits vorgeschlagen, eine rasche Determinierung von Agrobacterium vitis durch DNA-Hybridisierungen vorzunehmen. Um hierbei jedoch annähernd gleiche Spezifizität zu erreichen, muß die zu überprüfende DNA zunächst einigen Aufreinigungsschritten unterworfen werden. Zudem müssen jeweils gleiche DNA-Mengen auf die NC-Filter aufgetragen werden, um Signalstärken vergleichen zu können. Durch unterschiedliche Homologien der DNA-Sonden zu anderen Opin-Typen bzw. Biovaren von Agrobacterium tumefaciens ist jedoch die eindeutige Bestimmung von A. vitis nicht gewährleistet.

Es wurden auch Ganzzell-Fettsäure-Analysen zur Bestimmung von A. vitis eingesetzt. Es hat sich jedoch gezeigt, daß in nachteiliger Weise apathogene Isolate nicht von pathogenen unterschieden werden können, wobei zur sicheren Aussage zeitaufwendigere Pathogenitätstests erforderlich sind. Ein weiterer Nachteil liegt in der Notwendigkeit der Anreicherung der Bakterien auf Selektivmedien und der Erstellung von Reinkulturen, wobei bereits eine subjektive Vorauswahl getroffen werden muß und atypisch gewachsene bzw. Mischkolonien der Detektion entgehen können.

Schließlich wurde auch eine serologische Identifizierung von A. vitis mittels monoklonaler Antikörper durchgeführt. Der Nachweis von Agrobacterium tumefaciens mit Antiseren war jedoch bisher wegen des Vorkommens vieler verschiedener Serotypen für die Praxis ungeeignet. Bevor der Nachweis durchgeführt werden kann, muß eine Anreicherung der Bakterien auf Selektivmedien erfolgen, da für eine eindeutige Reaktion eine Mindestzellzahl von $2,3 \times 10^4$ Keimen erforderlich ist.

Angesichts dieser Problematik liegt der Erfindung die Aufgabe zugrunde, ein Verfahren zur Verfügung zu stellen, mittels welchem eine Bestimmung von Agrobacterium vitis rasch und eindeutig möglich ist, um einen Befall bzw. eine Infektion frühzeitig erkennen zu können, zur Einleitung von Behandlungsverfahren oder Selektion von Vermehrungsmaterial.

Gelöst wird diese Aufgabe gemäß der Erfindung dadurch, daß spezifische DNA-Fragmente von Tumorgenen und/oder Insertionselementen mittels der Polymerase-Kettenreaktion amplifiziert und anschließend durch DNA-Gelelektrophorese identifiziert werden.

Hierdurch läßt sich überraschend schnell und eindeutig eine Determinierung von Agrobacterium vitis durchführen.

Polymerase-Ketten-Reaktion (PCR) ist die Bezeichnung für ein in der Gentechnologie angewandtes Verfahren zur gezielten Vervielfältigung eines spezifischen DNA-Fragments. Hierbei kann eine bestimmte DNA-Sequenz unter einer Vielzahl ähnlicher Sequenzen erkannt und in vitro in relativ kurzer Zeit mengenmäßig stark angereichert und damit der Analyse zugänglich gemacht werden. Die PCR basiert darauf, daß drei Reaktionsschritte vielfach wiederholt ablaufen. Bei jedem Durchlauf eines Zyklus (Aufheizen, Abkühlen, Polymerisation) wird die Menge des Gens verdoppelt. Damit ist nach einer relativ kurzen Zeit eine Identifizierung durch DNA-Elektrophorese möglich.

Nach einer bevorzugten Ausführungsform des erfindungsgemäßen Verfahrens amplifiziert man spezifische Insertionselemente des TI-Plasmids. Zusätzlich unterwirft man für den Direktnachweis in Pflanzenextrakt aus inokulierten Weinreben die isolierte Bakterien-DNA einer Phenolextraktion und Ethanolpräzipitation.

Das erfindungsgemäße Verfahren wird nachfolgend anhand von Beispielen und der Zeichnung näher erläutert. In dieser zeigen:

Fig. 1        eine Darstellung der T-Region von A. vitis-Genomen mit Kennzeichnung der amplifizierten Bereiche;

Fig. 2        eine Darstellung zur Veranschaulichung des Nachweises von A. vitis-Octopin-Stämmen durch Amplifizierung spezifischer DNA-Fragmente;

Fig. 3        eine Darstellung zur Veranschaulichung des Nachweises von A. vitis-Vitopin-Stämmen durch Amplifizierung spezifischer DNA-Fragmente;

Fig. 4 eine Darstellung zur Veranschaulichung des Nachweises von A. vitis-Nopalin-Stämmen durch Amplifizierung spezifischer DNA-Fragmente;

Fig.5a,5b Darstellungen zur Unterscheidung verschiedener A. vitis-Genomtypen anhand des Spektrums an IS 866, IS 868 und IS 869; und

Fig. 6 eine weitere Darstellung der T-Region von A. vitis-Genomen mit Kennzeichnung der amplifizierten Bereiche.

Beispiel 1:

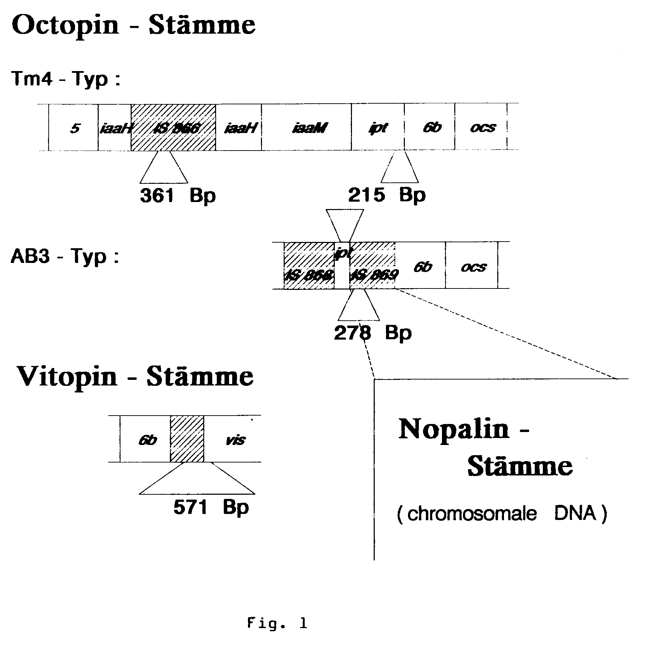

Oligonukleotideprimer mit einem Gehalt von 19 bis 26 Nukleotiden wurden ausgewählt, um spezifische Fragmente zu amplifizieren. Die Primermerkmale sind in Tabelle 1 dargestellt. Die Fig. 1 enthält eine physikalische Darstellung des ausgewählten T-DNA-Bereichs und der amplifizierten Fragmente. Die verwendeten A. vitis-Stämme mit Isolationstyp und Herkunft sind in Tabelle 2 dargestellt.

Bei der DNA-Isolation wurde die Spezifizität von den selektierten Primern untersucht.

**Auswahl der zu amplifizierenden DNA-Fragmente:**

Anhand der T-DNA-Struktur verschiedener Agrobacterium vitis Ti-Plasmide (Fig. 1) werden die in Tabelle 1 aufgelisteten Oligonukleotide synthetisiert, die den spezifischen Nachweis der in Fig. 1 gekennzeichneten Ti-Plasmid-Regionen ermöglichen.

**DNA Isolierung aus Bakterienreinkulturen:**

Kolonien der auf Nährboden angereicherten Bakterienkulturen werden mit Hilfe einer Impföse aufgenommen, in ein Eppendorf-Reaktionsgefäß transferiert und in 1 ml PBS resuspendiert. Die Suspension wird kurz abzentrifugiert, der Über-stand verworfen und das Pellet in 0,5 ml destilliertem Wasser aufgenommen. Die optische Dichte der Bakteriensuspension wird bestimmt und ein Aliquot entsprechend 0,1 OD in 0,5 ml Reaktionsgefäße transferiert. Die Reaktionsansätze werden auf ein Endvolumen von 50 $\mu$l mit PCR-Puffer eingestellt und 10 Minuten bei 95 °C inkubiert.

**Polymerase Ketten-Reaktion:**

Nach Abkühlung der Gefäße auf Raumtemperatur werden 400 $\mu$M Primer-Oligonukleotide, 200 $\mu$M Nukleotide und 2,5 Einheiten TAQ-Polymerase zugegeben und mit 2 Tropfen sterilem Paraffin überschichtet. Nach erneuter Inkubation bei 94 °C für 2 Minuten erfolgt die Amplifizierung durch Fahren von 30 Temperaturzyklen wie folgt:

1. 30 Sekunden bei 94 °C
2. 30 Sekunden bei 50 °C
3. 30 Sekunden bei 72 °C.

Temperaturstufe 3 wird bei jedem Zyklus um 3 Sekunden verlängert, um die Verschlechterung der Reaktionsbedingungen mit fortschreitender Reaktionsdauer zu kompensieren.

Die amplifizierten DNA-Fragmente werden anschließend durch Gelelektrophorese in 2%igem Agarosegel mit 0,5 $\mu$g/ml Ethidiumbromid visualisiert.

**Probenvorbereitung für die Untersuchung von Pflanzen auf Infektion mit A. vitis:**

Für den Nachweis von A. vitis in Pflanzen werden 0,5 g des Pflanzenmaterials zerkleinert und in flüssigem Stickstoff pulverisiert. Das gefrorene Pulver wird in Eppendorf-Reaktionsgefäße transferiert und 1 ml Nährlösung zugegeben. Anschließend werden die Ansätze zwei Stunden bei 28 °C inkubiert, wobei sich verbleibende Pflanzenreste absetzen. Die Konzentration von A. vitis-Zellen kann durch Ausplattierung eines Teils des Überstandes auf Selektivmedium bestimmt werden.

Der verbleibende Überstand wird in neue Reaktionsgefäße überführt und die Bakterien-DNA nach Standard-DNA-Miniprep Protokollen isoliert. Die isolierte DNA wird anschließend einer Phenolextraktion und Ethanol-Fällung unterworfen und in 50 $\mu$l TE-Puffer aufgenommen. Der DNA-Gehalt wird photometrisch bestimmt und 200 ng für die Durchführung der Amplifizierung verwendet. Die Amplifizierung und Visualisierung erfolgt wie bereits beschrieben.

**Ergebnisse und Diskussion:**

1. Identifizierbare A. vitis-Stämme:

a) Octopin/Cucumopin-Stämme:

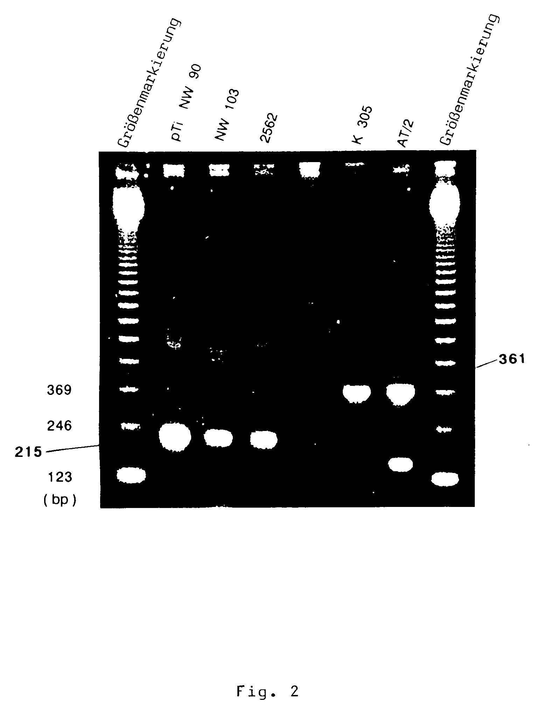

Octopin-Cucumopin-Stämme werden identifiziert anhand eines Fragments des Tm4-ipt Gens. Der Nachweis von A. vitis-Octopin-Stämmen anhand des Tm4-ipt-Gens (NW 90, NW 103, 2562) und/oder IS 866 (K 305, AT-2) ist in Fig. 2 veranschaulicht.

Desweiteren können Stämme dieses Typs mit breitem Wirtsspektrum (WHR-Stämme) von Stämmen mit engem Wirtsspektrum (LHR-Stämme) anhand des vorhandenen Spektrums der Insertionselemente IS 866 und IS 868 unterschieden werden.

b) Vitopin-Stämme:

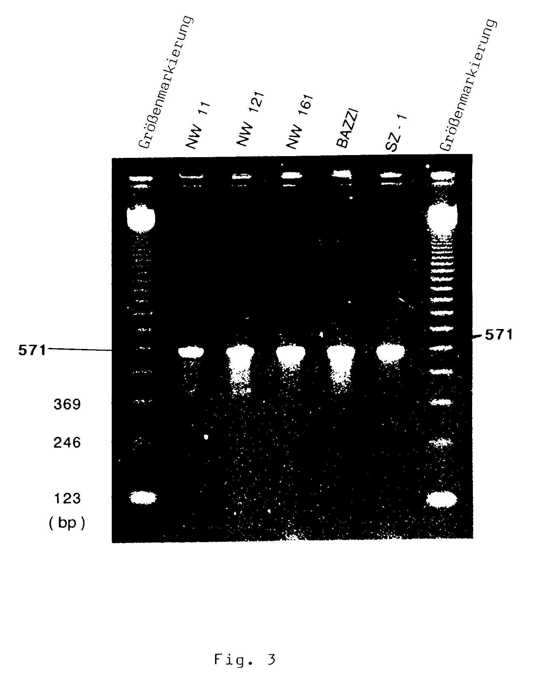

Vitopin-Stämme werden durch Amplifizierung eines Fragments aus der 6b/vis-Region der Vitopin-T-DNA identifiziert (Fig. 3). Zusätzlich können zwei verschiedene Genomgruppen dieser Stämme anhand des Nachweises der Insertionselemente IS 868 und IS 869 unterschieden werden.

c) Nopalin-Stämme:

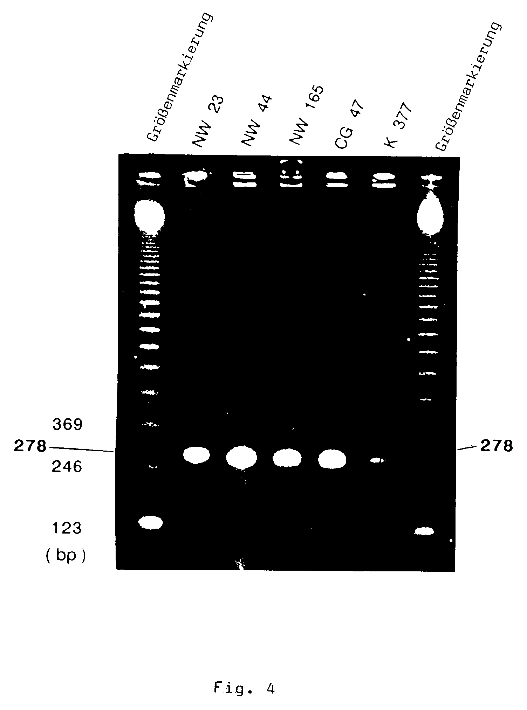

Nopalin-Stämme werden durch den Nachweis des Insertionselements IS 869 bei gleichzeitiger Abwesenheit aller anderen o.g. molekularen Marker identifiziert (Fig. 4).

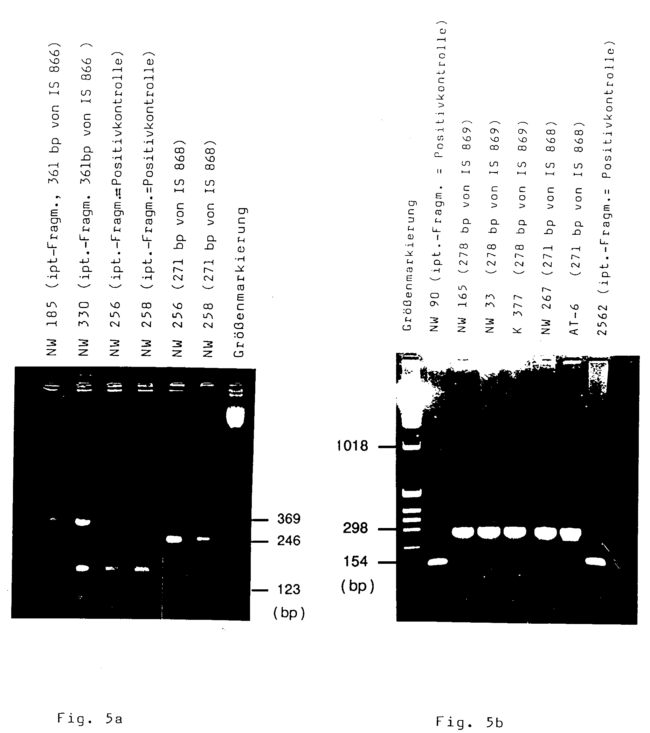

Wie aus den Fig. 5a, 5b ersichtlich, können verschiedene A. vitis-Genomtypen anhand des Spektrums an IS 866, IS 868 und IS 869 unterschieden werden. Hierbei wurde ein 149 Bp großes Fragment des Tm4-ipt Gens als Positivkontrolle verwendet. Die Unterscheidung verschiedener Genomtypen innerhalb A. vitis ermöglicht eine Rückverfolgung des Infektionsweges.

2. Nachweis von A. vitis in Pflanzen:

Für den Direktnachweis von A. vitis in Pflanzen muß die isolierte Bakterien-DNA zusätzlichen Reinigungsschritten unterworfen werden, damit eine Amplifizierung erreicht werden kann. Dies kann durch Phenolextraktion und Ethanolfällung in ausreichendem Maßstab erfolgen.

3. Vorteile gegenüber herkömmlichen Verfahren:

a) Die Identifizierung von A. vitis nach Anreicherung auf Nährböden kann innerhalb eines halben Tages erfolgen, wobei zusätzlich der zugehörige Genomtyp erkannt wird. Dadurch ist es möglich, detaillierte Aussagen über die Art der Infektion zu treffen. Eine Isolierung und Reinigung bakterieller DNA ist bei Anwendung dieses Verfahrens nicht notwendig.

b) Durch Isolierung und Reinigung bakterieller DNA aus Pflanzen kann der Direktnachweis der Bakterien innerhalb zweier Arbeitstage erfolgen.

c) Durch die gleichzeitig erfolgende detaillierte Identifizierung verschiedener Genomtypen ist es möglich, Aussagen über Infektionswege zu treffen.

Beispiel 2:

**Auswahl der zu amplifizierenden DNA-Fragmente:**

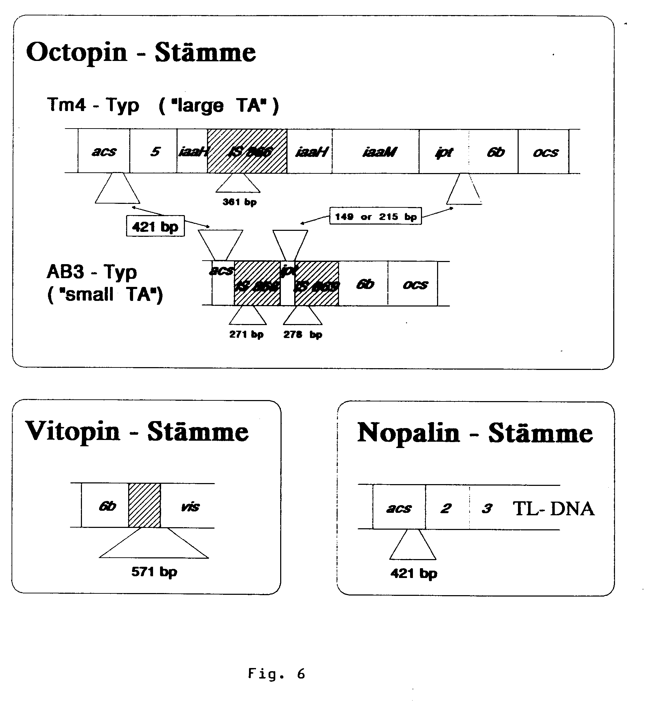

Zu den im Zusammenhang mit Beispiel 1 beschriebenen Bereichen der T-DNA verschiedener Agrobacterium vitis Ti-Plasmide werden Oligonukleotide für das Tm4-acs Gen selektiert, welches in Octopin- und Nopalin-Stämmen nachgewiesen und sequenziert wurde (Tabelle 3). Die Lokalisierung des acs-Gens ist in Fig. 6 für die T-DNA-Region von A. vitis Octopin- und Nopalin-Stämme dargestellt.

**Probenvorbereitung für den molekularbiologischen Nachweis:**

Kolonien der auf Nährböden angereicherten Bakterienkulturen werden mit Hilfe einer Impföse aufgenommen, in ein Eppendorf-Reaktionsgefäß transferiert und in 1 ml sterilem PBS resuspendiert. Die Suspension wird 3 - 5 Minuten abzentrifugiert, der Überstand verworfen und das Pellet in 1 ml sterilem bidestilliertem Wasser aufgenommen. Dieser Vorgang wird wiederholt. Nach der Zentrifugation werden die Bakterien in 200 $\mu$l PCR-Puffer aufgenommen und 10 Minuten bei 95°C lysiert. Nach Abkühlung der Gefäße auf Raumtemperatur werden entsprechende Aliquots (z.B. 18,5 $\mu$l für 25 $\mu$l Ansätze) verwendet.

**Polymerase Ketten-Reaktion:**

Zusätzlich zu dem Verfahren gemäß Beispiel 1 werden für den Komplettnachweis Oligonukleotide sowohl für die Detektion des Tm4-acs als auch des S4-6b/vis-Gens in einen Reaktionsansatz gegeben. Das weitere Verfahren entspricht dem Beispiel 1.

**Ergebnisse und Diskussion:**

1. Identifizierbare A. vitis-Stämme:
   a) Komplettnachweis
   Der Nachweis von Agrobacterium vitis erfolgt durch die Amplifizierung entweder des Tm4-acs- oder des S4-6b/vis-Genfragments angezeigt durch eine DNA-Bande von 421 bp ( = Tm4-acs) oder von 571 ( = S4-6b/vis).
   Die weitere Identifizierung (falls notwendig oder erwünscht) erfolgt entsprechend dem Verfahren gemäß Beispiel 1.

**Vorteile gegenüber herkömmlichen Verfahren:**

Zusätzlich zu den in Zusammenhang mit Beispiel 1 beschriebenen Vorteilen können durch Verwendung des Tm4-acs-Gens in Kombination mit dem S4-6b/vis-Gen sämtliche bislang bekannte Agrobacterium vitis-Stämme **in einem Reaktionsansatz** identifiziert werden. Durch gleichzeitige Reduktion des Reaktionsansatzes auf 25µl können die Kosten für den Nachweis wesentlich reduziert werden.

Zusammenfassend ist festzustellen, daß das erfindungsgemäße Verfahren ein empfindliches Diagnoseverfahren für den Nachweis des Maukeerregers Agrobacterium vitis sp. nov. in latent infizierten Weinreben darstellt. Die Methode beruht auf der Amplifizierung von Tumorfragmenten und spezifischen Isertionselementen des Ti-Plasmids mittels der Polymerase-Kettenreaktion (PCR). Für den Direktnachweis in Pflanzenextrakten aus inokulierten Weinreben wird die isolierte Bakterien-DNA zusätzlich einer Phenolextraktion und Ethanolpräzipitation unterworfen.

Die Empfindlichkeit des Testsystems liegt zur Zeit bei ca. 200 A. vitis-Bakterien pro ml Pflanzenextrakt bei infizierten Versuchspflanzen der Art Kalanchoe daigremontiana. Bei Extrakten aus Weinreben waren positive Reaktionen nicht reproduzierbar.

Zusätzlich zur speziesspezifischen Identifizierung können mit dieser Methode gleichzeitig verschiedene Genomgruppen innerhalb A. vitis unterschieden werden, wodurch eine Rückverfolgung des Infektionsweges erstmalig möglich gemacht wird.

Tabelle 1

| Oligonucleotide für die Amplifizierung *A. vitis* − spezifischer DNA-Fragmente | | |
|---|---|---|
| | Primer 1 | Primer 2 |
| 361 Bp von *IS 866* | 5'-ACTTTCGCTGTGGTCATCAGG-3' | 3'-CGAAGCCAAACGATACTTCTG-5' |
| 271 Bp von *IS 868* | 5'-CTGGCGGTATATTCCCAATCG-3' | 3'-CGCTAGGCTTACTACGTGTTC-5' |
| 278 Bp von *IS 869* | 5'-CCAAATTCTTACTGCTGCC-3' | 3'-CGTGGGAACCTCTAACAGACAT-5' |
| 149 Bp of Tm4-*ipt* | 5'-TTGCAGCTTGACGCAGATATGG-3' | 3'-CGGGTGGTAAGCCTCGCATGAT-5' |
| 215 Bp von Tm4-*ipt* | 5'AGATCGATGGATATCGATATGCCAT3' | 3'TCCGGGTGGTAAGCCTCGCATGATC-5' |
| 571 bp von *vis/6b* | 5'-TTCTGGTGTAAGTGCGACATATT-3' | 3'CCACTACGTAACAACACCCACAGGGC5' |

Tabelle 2. Verwendete A. *vitis*-Stämme

| Stamm | Opin-typ[a] | Isolation[b] | Herkunft[c] |
|---|---|---|---|
| NW 1 | NOP | Scheurebe (Wr) | BRD (NW) |
| NW 11 | VIT | Riesling (Wr) | BRD (NW) |
| NW 22 | NOP | Müller-Th (Wr) | BRD (NW) |
| NW 23 | NOP | Müller-Th (Wr) | BRD (NW) |
| NW 33 | VIT | Kerner (Wr) | BRD (NW) |
| NW 44 | NOP | Morio-Muskat (Wr) | BRD (NW) |
| NW 90 | OCT | Riesling (Wr) | BRD (NW) |
| NW 102 | OCT | Riesling (Wr) | BRD (NW) |
| NW 103 | OCT | Riesling (Wr) | BRD (NW) |
| NW 113 | VIT | Müller-Th (Wr) | BRD (NW) |
| NW 121 | VIT | Riesling (Wr) | BRD (NW) |
| NW 150 | NOP | n.b. (Wr) | BRD (NW) |
| NW 160 | NOP | Scheurebe (Wr) | BRD (NW) |
| NW 161 | VIT | Scheurebe (Wr) | BRD (NW) |
| NW 163 | NOP | Riesling (Wr) | BRD (NW) |
| NW 165 | NOP | Scheurebe (Wr) | BRD (NW) |
| NW 170 | NOP | Riesling (Wr) | BRD (NW) |
| NW 180 | OCT | Riesling (Wr) | BRD (NW) |
| NW 183 | OCT | Scheurebe (Wr) | BRD (NW) |
| NW 185 | OCT | Riesling (Wr) | BRD (NW) |
| NW 190 | NOP | Portugieser (Wr) | BRD (NW) |
| NW 256 | VIT | n.b. (Wr) | BRD (NW) |
| NW 258 | VIT | n.b. (Wr) | BRD (NW) |
| NW 263 | VIT | n.b. (Wr) | BRD (NW) |
| NW 267 | VIT | n.b. (Wr) | BRD (NW) |
| NW 310 | NOP | n.b. (Wr) | BRD (NW) |
| NW 321 | VIT | n.b. (Wr) | BRD (NW) |
| NW 330 | OCT | n.b. (Wr) | BRD (NW) |
| NW 970 | VIT | n.b. (Wr) | BRD (NW) |
| NW 1100 | NOP | n.b. (Wr) | BRD (NW) |
| NW 1500 | NOP | n.b. (Wr) | BRD (NW) |
| NW 12556 | OCT | n.b. (Wr) | BRD (NW) |
| NWT 11/72 | VIT | Gewürztram. (Wr) | BRD (NW) |

**Tabelle 2** (Fortsetzung)

| Stamm | Opin-typ[a] | Isolation[b] | Herkunft[c] | |
|-------|------------|-------------|-------------|---|
| AT-6 | OCT | Weinrebe | Ungarn | (ES) |
| Sz-1 | VIT | Weinrebe | Ungarn | (ES) |
| BAZZI | VIT | Weinrebe | Italien | (CB) |
| K 305 | OCT | Weinrebe | Australien | (AK) |
| K 377 | OCT | Weinrebe | Australien | (AK) |
| CG 42 | NOP | Weinrebe | USA | (TB) |
| CG 47 | NOP | Weinrebe | USA | (TB) |
| 12/1 | – | Weinrebe | Süd Afrika | (JS) |

[a]NOP, Nopalin; OCT, Octopin; VIT, Vitopin, n.b., nicht bekannt

[b]Wr, Weinrebe

[c]NW, Stammsammlung der LLFA Neustadt/Weinstr., ES, E. Szegedi;
CB, C. Bazzi; AK, A.Kerr; TB, T. Burr; HP, H.J. Du Plessis;
EN, E.W. Nester; CK, C.I. Kado; JS, J.L. Staphorst;
ATCC, American Type Culture Collection, Rockville, USA;
NCPPB, National Collection of Plant Pathogenic Bacteria,
Harpenden, Great Britain.

Tabelle 3

| Oligonukleotide für die Amplifizierung *A.vitis*-spezifischer DNA-Fragmente | | |
|---|---|---|
| Fragment | Primer 1 | Primer 2 |
| 361 Bp von Tm4 IS*866* | 5'-ACTTTCGCTGTGGTCATCAGG-3' | 3'-CGAAGCCAAACGATACTTCTG-5' |
| 271 Bp von AB3 IS*868* | 5'-CTGGCGGTATATTCCCAATCG-3' | 3'-CGCTAGGCTTACTACGTGTTC-5' |
| 278 Bp von AB3 IS*869* | 5'-CCAAATTCTTACTGCTGCC-3' | 3'-CGTGGGAACCTCTAACAGACAT-5' |
| 149 Bp von Tm4 *ipt* | 5'-TTGCAGCTTGACGCAGATATGG-3' | 3'-CGGGTGGTAAGCCTCGCATGAT-5' |
| 215 Bp von Tm4 *ipt* | 5'-AGATCGATGGATATCGATATGCCAT-3' | 3'-TCCGGGTGGTAAGCCTCGCATGATC-5' |
| 571 Bp von S4 *6b/vis* | 5'-TTCTGGTGTAAGTGCGACATATT-3' | 3-'CCACTACGTAACAACACCCACAGGGC-5' |
| 421 Bp von Tm4 *acs* | 5'-CGA GAA TTA TAG ACG GAT CGC-3' | 3'-GGACGGACACAATTACAGCTA-5' |

EP 0 603 881 A2

**Patentansprüche**

1. Verfahren zum Nachweis von Agrobacterium vitis in Weinreben, wobei T-DNA-Fragmente des Pathogens in infizierten Weinreben mittels Polymerase-Kettenreaktion (PCR) nachgewiesen werden,
**dadurch gekennzeichnet,**
daß spezifische DNA-Fragmente von Tumorgenen und/oder Insertionselementen mittels der Polymerase-Kettenreaktion amplifiziert und anschließend durch DNA-Gelelektrophorese identifiziert werden.

2. Verfahren nach Anspruch 1, dadurch gekennzeichnet, daß der Nachweis mit Bakterienreinkulturen ohne zusätzliche DNA-Isolierung durchgeführt wird, indem die Bakterien in einem Reaktionspuffer lysiert und anschließend die DNA-Fragmente amplifiziert werden.

3. Verfahren nach Anspruch 1, dadurch gekennzeichnet, daß die isolierte DNA aus Pflanzenextrakten (bakterielle und/oder pflanzliche DNA) einer Phenolextraktion und Ethanolpräzipitation unterworfen wird.

# Octopin - Stämme

## Tm4 - Typ :

| 5 | iaaH | IS 866 | iaaH | iaaM | ipt | 6b | ocs |

**361 Bp**  **215 Bp**

## AB3 - Typ :

| IS 866 ipt IS 869 | 6b | ocs |

**278 Bp**

# Vitopin - Stämme

| 6b | | vis |

**571 Bp**

# Nopalin - Stämme

( chromosomale DNA )

Fig. 1

Fig. 2

Fig. 3

Fig. 4

12

Fig. 5a

NW 185 (ipt-Fragm., 361 bp von IS 866)
NW 330 (ipt.-Fragm. 361bp von IS 866 )
NW 256 (ipt.-Fragm.≅Positivkontrolle)
NW 258 (ipt.-Fragm.=Positivkontrolle)
NW 256 (271 bp von IS 868)
NW 258 (271 bp von IS 868)
Größenmarkierung

369
246
123
(bp)

Fig. 5b

Größenmarkierung
NW 90 (ipt.-Fragm. = Positivkontrolle)
NW 165 (278 bp von IS 869)
NW 33 (278 bp von IS 869)
K 377 (278 bp von IS 869)
NW 267 (271 bp von IS 868)
AT-6 (271 bp von IS 868)
2562 (ipt.-Fragm.= Positivkontrolle)

1018
298
154
(bp)

Fig. 6